# FASCICULE DE BREVET EUROPEEN

(11) **EP 2 989 194 B1**
(45) Date de publication et mention de la délivrance du brevet: **25.01.2017**
(21) Numéro de dépôt: 14722263.2
(22) Date de dépôt: 11.04.2014
(51) Int. Cl.: C12M 1/00, C12M 1/06

(54) **RÉACTEUR À ÉCLAIRAGE INTÉGRÉ**
REAKTOR MIT INTEGRIERTER BELEUCHTUNG
REACTOR WITH INTEGRATED ILLUMINATION

(30) Priorité: 22.04.2013 FR 1353641
(43) Date de publication de la demande: 02.03.2016
(73) Titulaire: Fermentalg, 33500 Libourne (FR); PIERRE GUERIN S.A., 79210 Mauze sur le Mignon (FR)
(72) Inventeur: GARNIER, Patrice, F-33230 Lagorce (FR); GODART, François, F-33870 Vayres (FR); CALLEJA, Pierre, 33500 Libourne (FR); GIRAUD, Samuel, F-17000 La Rochelle (FR); FELEZEU, Doru, F-75013 Paris (FR)
(74) Mandataire: Be IP
(86) Numéro de dépôt international: PCT/FR2014/050891
(87) Numéro de publication internationale: WO 2014/174182

(56) Documents cités:
- WO-A1-2008/104599
- WO-A1-2010/089151
- US-A1- 2009 134 173
- US-A1- 2010 005 711
- US-A1- 2010 311 156
- DATABASE WPI Week 201047 Thomson Scientific, London, GB; AN 2010-H69901 XP002716662, & CN 201 501 874 U (CHANGZHOU SUNGOD BIOTECHNOLOGY & ENG EQU) 9 juin 2010 (2010-06-09)

## Description

L'invention concerne un réacteur à éclairage intégré, notamment adapté à la culture de micro-organismes photosensibles. Il peut s'agir d'un bioréacteur mais aussi d'un réacteur chimique ou physico-chimique.

La notion de bioréacteur, ou réacteur biologique, désigne ici un réacteur au sein duquel se développent des phénomènes biologiques, tels qu'une croissance de cultures de micro-organismes pures ou d'un consortium de micro-organismes (notamment des micro-algues), dans des domaines très variés tels que le traitement d'effluents, la production de biomasse contenant des biomolécules d'intérêt (c'est-à-dire des biomolécules que l'on sait valoriser). Cette notion englobe donc notamment les réacteurs appelés fermenteurs.

Un réacteur comporte typiquement une cuve fermée au sein de laquelle est monté un élément de brassage ou d'agitation destiné à favoriser une homogénéisation du contenu de la cuve ; un tel élément de brassage est habituellement constitué d'un arbre vertical portant des pales ou des turbines dont le mouvement, au sein de la masse en cours de traitement dans le réacteur, assure le brassage et son homogénéité.

Divers types de conditions opératoires peuvent être nécessaires pour la croissance des espèces biologiques au sein d'un tel bioréacteur ou fermenteur ; on connaît ainsi, notamment, des régimes de croissance hétérotrophe (avec des apports de source carbonée tels que les sucres), de croissance autotrophe (ou photo-autotrophe) avec un apport de lumière (on parle aussi de photosynthèse) ou de croissance mixotrophe (avec un apport combiné de source carbonée et de lumière). Une approche scientifique de ce sujet est abordée notamment dans le document « Astaxanthin production by Haematococcus pluvialis under illumination with LEDs » de Katsuda et al, paru dans Enzyme and Microbial Technology 35 (2004) 81-86, ou dans le document « Effects of using light-emitting diodes on the cultivation of Spirulina platensis » de Wang et al, paru dans Biochemical Engineering Journal 37 (2007) 21-25.

Diverses configurations ont déjà été proposées pour effectuer un tel apport de lumière, en continu ou de manière cyclique (avec des cycles dont la durée peut varier de quelques minutes à plusieurs heures), voire sous forme d'impulsions à la manière de flash, avec un spectre approchant de celui de la lumière du jour ou au contraire étroitement centré sur une longueur d'onde choisie. Des réacteurs adaptés à un tel apport de lumière sont parfois appelés photoréacteurs ou photobioréacteurs (lorsqu'ils sont le lieu de traitements biologiques).

Une configuration bien maîtrisée consiste à munir la cuve de hublots, par exemple entre six et douze selon la taille de la cuve, permettant la pénétration d'une lumière générée depuis l'extérieur de la cuve. De tels hublots peuvent avoir de 5 à 20 cm de diamètre en pratique. Un inconvénient d'une telle configuration est que les hublots limitent la surface d'illumination et absorbent ou réfléchissent une partie significative des photons émis par la source d'éclairage.

C'est pourquoi d'autres configurations ont été proposées dans lesquelles des sources de lumières sont implantées au sein de la cuve.

Ainsi, le document WO - 2002/086053, après avoir mentionné le cas de cuves munies de hublots, décrit un ensemble comportant une cuve de croissance, une cuve d'illumination en communication avec la cuve de croissance et munie d'une source de lumière, de préférence située à l'intérieur de cette cuve d'illumination, avec un système de mise en circulation de micro-organismes d'une cuve à l'autre ; la cuve d'illumination est en pratique tubulaire et comprend des déflecteurs pour assurer que l'écoulement des micro-organismes soit turbulent ; la source de lumière est de préférence concentrique à la cuve d'illumination et peut être constituée d'une lampe à halogénure métallique, par exemple une lampe au sodium et à haute pression. Il est recommandé que la cuve de croissance comporte également une source de lumière, assurant une illumination au travers de hublots que comporte la paroi de la cuve ou formée de tubes de verre immergés dans la masse de micro-organismes.

Le document US - 2010/0190227 (découlant de la demande de brevet international WO - 2008/145719) concerne un bioréacteur comportant des pièces en matière plastique moulées dans lesquelles des diodes électroluminescentes (LED en abrégé) sont intégrées ; il est envisagé que ces pièces soient des plaques ou bandes lorsqu'il s'agit d'équiper les parois internes de la cuve, mais il est recommandé que ces pièces soient tubulaires, de préférence en faisceaux, lorsque ces sources lumineuses sont disposées à l'intérieur du réacteur, ainsi que cela ressort des exemples considérés, auquel cas les diodes sont alimentées au travers du couvercle de la cuve. Les diodes LED émettent par exemple dans le domaine du rouge. La matière plastique dans laquelle elles sont implantées peut être de la silicone.

Par ailleurs, le document US - 7 824 904 décrit un réacteur dont le dispositif d'agitation comporte des pales portant des sources de lumière telles que des lampes, des ampoules ou des LED.

Quant au document WO - 2011/154886, il décrit de même un réacteur comportant un système d'illumination solidaire d'un ensemble tournant.

Il ressort de ces divers documents que le fait de générer la lumière à l'extérieur de la cuve et de la faire pénétrer au sein de la cuve est généralement considéré comme ne présentant pas une efficacité suffisante et qu'il est très généralement estimé qu'une bonne efficacité implique de disposer des sources lumineuses au sein même de la masse contenue dans la cuve, de préférence sur l'ensemble tournant qui y est disposé ; diverses solutions sont proposées pour la connexion de ces sources avec l'extérieur.

Il mérite d'être noté que l'utilité d'une illumination au sein d'un réacteur ne se limite pas à des traitements biologiques ; ainsi, un rayonnement UV peut être utile pour stériliser des milieux à traiter (voir par exemple le document US - 4,456,512) ; il y a en outre des cas où un rayonnement de lumière visible ou non peut favoriser des réactions chimiques ou des traitements physico-chimiques.

L'invention a pour objet un réacteur dans la cuve duquel des sources lumineuses sont disposées d'une manière permettant une illumination efficace d'une masse à traiter contenue dans la cuve tout en minimisant les modifications apportées à cette cuve pour l'intégration de ces sources lumineuses.

L'invention propose à cet effet un réacteur comportant une cuve destinée à contenir une masse à traiter et munie d'une part d'un ensemble tournant autour d'un axe destiné à assurer un brassage de cette masse à traiter et d'autre part d'une pluralité de sources d'illumination destinées à favoriser le traitement de cette masse, cette cuve ayant une paroi interne à laquelle sont fixées des plaques dont les plans sont orientés vers l'axe de rotation de l'ensemble tournant, et parallèlement à celui-ci, en sorte d'empêcher la formation d'un vortex au sein de la masse à traiter sous l'action de l'ensemble tournant, ces sources d'illumination étant portées par lesdites plaques en étant encapsulées, avec au moins la partie de ces plaques qui les porte, dans une matière compatible avec la masse à traiter et en une épaisseur permettant de diffuser ladite lumière vers l'intérieur de la cuve.

On comprend que la masse à traiter est liquide, pâteuse ou pulvérulente, c'est-à-dire qu'elle a une fluidité suffisante pour pouvoir être brassée. Il peut s'agir d'une masse de micro-organismes dont on veut, par exemple, favoriser la croissance et/ou l'enrichissement ; mais cette masse peut aussi être une masse très fluide, ou au contraire granulaire, dont on veut par exemple assurer une bonne stérilisation.

Ainsi l'invention tire profit de ce qu'un certain nombre de cuves de réacteur comportent des plaques s'étendant depuis leur paroi interne vers le centre de la cuve tout en restant à distance de l'ensemble tournant (les cuves sont habituellement cylindriques de sorte que ces plaques sont orientées radialement) ; de telles plaques sont souvent appelées contre-pales, étant destinées à éviter l'apparition de vortex à l'intérieur de la cuve en cas de brassage très turbulent effectué par l'ensemble tournant, qui est habituellement muni de pales. Le fait que les sources d'illumination sont portées par de telles plaques permet un bien meilleur rapport S/V (surface d'illumination par rapport au volume de la cuve) que dans les solutions connues et mises en oeuvre.

Ainsi, la configuration de l'invention permet une meilleure efficacité de l'illumination par rapport à une configuration où la lumière pénètre par des hublots à partir de sources disposées à l'extérieur ; par ailleurs, les contre-pales offrent une surface de support bien supérieure que dans les configurations où les sources d'illumination sont disposées sur l'ensemble tournant ou sur des pièces tubulaires plongeant dans la masse à traiter, ou encore au fond ou au plafond de la cuve. En outre, la configuration de l'invention permet d'obtenir un rapport S/V dont l'ordre de grandeur peut être conservé lorsqu'on augmente la taille de la cuve ; ainsi, si on augmente la hauteur et deux dimensions transversales de la cuve dans un rapport R et si on conserve le rapport entre la dimension transversale des plaques par rapport aux dimensions transversales de la cuve et celui entre la hauteur de ces plaques par rapport à la hauteur des cuves, le rapport S/V devient S/VR (un doublement du volume de la cuve n'induit qu'une baisse de 20% du rapport S/V ; inversement, il suffit d'augmenter le rapport entre la dimension transversale des plaques et celle de la cuve de 20% pour conserver le rapport S/V tout en doublant le volume de la cuve) ; en jouant sur le nombre de plaques portant les sources d'illumination, on peut noter que si on décuple le volume d'une cuve, on peut conserver au moins approximativement le rapport S/V en se contentant de doubler le nombre de plaques tout en conservant le rapport entre les dimensions de ces plaques et celles de la cuve. Un concepteur de cuve peut donc assez facilement, à partir d'une cuve donnée, extrapoler les dimensions à donner à une cuve de plus grandes dimensions pour obtenir un niveau de performances donné. La configuration de l'invention a donc pour avantage de faciliter la conception de cuves de plus en plus grandes à partir de cuves plus petites, par exemple des cuves ayant servi de prototypes.

Il est à noter que le fait que les sources d'illumination sont situées à proximité de la paroi interne de la cuve (puisqu'elles la longent par une tranche) rend plus simple la connexion des sources avec l'extérieur, par rapport au cas d'une implantation dans la masse à traiter, par exemple sur l'ensemble tournant.

Le fait que les sources d'illumination sont portées par des plaques statiques par rapport à la cuve évite de modifier l'encombrement des éléments tournants, leur inertie ou la puissance nécessaire pour leur mouvement.

En fait, il est apparu que, contrairement aux préconisations de divers documents antérieurs, le fait de disposer les sources d'illumination à l'écart de la portion centrale de la cuve permet d'obtenir une grande puissance d'illumination par unité de volume sans avoir à utiliser des sources d'illumination très puissantes ; il suffit d'implanter un nombre suffisant de sources d'illumination même si leur puissance individuelle est modérée ; la configuration de l'invention permet donc une bonne illumination pour un coût d'installation plus faible que dans les solutions connues. En pratique, le brassage assuré par l'ensemble tournant suffit à garantir que l'ensemble de la masse à traiter tire profit de l'illumination. Toutefois, le fait que les sources d'illumination sont disposées sur les contre-pales et non sur la paroi interne de la cuve optimise la fraction de la masse à traiter venant au cours du brassage à proximité de ces sources d'illumination.

Un autre avantage de disposer les sources d'illumination sur les plaques servant de contre-pales est de maximiser la surface disponible pour porter de telles sources d'illumination ; en effet, la pluralité de sources d'illumination peut comporter des sources d'illumination distribuées sur chacune des faces desdites plaques.

Les plaques portant les sources d'illumination peuvent être fixes.

Toutefois, de manière avantageuse, les plaques portant les sources d'illumination sont démontables vis-à-vis de la paroi de la cuve, donc extractibles hors de la cuve. Un avantage est qu'ainsi, les opérations de maintenance de ces plaques et de ces sources d'illumination, ainsi que celles de la cuve, sont simplifiées par rapport à une configuration où ces sources sont portées par la paroi interne. Un autre avantage est qu'une même cuve peut être équipée de diverses configurations d'illumination (il suffit de disposer de plusieurs jeux de contre-pales comportant un nombre différent et/ou une nature différente de sources individuelles d'illumination) ; le fait de disposer plusieurs jeux de contre-pales comportant des configurations d'illumination différentes ou non permet en outre de minimiser les interruptions de fonctionnement lors des opérations de maintenance, puisqu'un jeu de contre-plaques peut être en maintenance pendant qu'un autre est en service ; de même, en cas de panne, la réparation peut se faire, de manière aisée, après s'être éventuellement contenté de remplacer les plaques par d'autres plaques munies d'une pluralité similaire de sources d'illumination.

De manière préférée, les sources d'illumination sont des diodes électro-luminescentes (LED en abrégé) ; il s'agit de sources de lumière bien maîtrisées, aussi bien dans leur mise en oeuvre que dans leurs applications. De telles sources peuvent avoir des spectres d'émission très divers, puisqu'il y a des LED blanches (simulant la lumière solaire), mais aussi des LED à gamme spectrale réduite (par exemple centrées sur une lumière rouge, bleue ou verte). De telles sources d'illumination génèrent moins de chaleur que des bulbes ou des ampoules ; elles ont en outre des dimensions suffisamment petites pour pouvoir être implantées sur les faces des contre-pales sans induire de surépaisseur nuisant à la fonction principale de ces plaques.

Le spectre des sources d'illumination est avantageusement dans le domaine du visible, mais peut aussi, en fonction des besoins, être à l'extérieur de ce domaine visible, par exemple dans les UV (par exemple pour des applications impliquant une stérilisation), ou dans les infra-rouges (par exemple, dans des applications visant à générer de la chaleur au sein de la masse à traiter).

Les sources d'illumination peuvent avoir des régimes de commande (on parle aussi de régime de contrôle ou de pilotage) très variés.

Ainsi, la pluralité de sources d'illumination peut être composée de plusieurs sous-ensembles, les sources d'illumination de chacun de ces sous-ensembles pouvant avoir un spectre spécifique d'émission. En complément ou en alternative, la pluralité de sources d'illumination est formée de plusieurs sous-ensembles ayant chacun un même régime d'excitation, en continu ou de manière cyclique, avec une intensité constante ou variable. En complément avec l'une ou l'autre des options précitées, ou en alternative, la pluralité de sources d'illumination est connectée à un ensemble de pilotage tel que certaines au moins des sources d'illumination peuvent être pilotées en mode d'éclairage ou non (c'est-à-dire qu'elles sont allumées ou éteintes selon les moments), en régime continu, en régime flash ou de manière cyclique, et en spectre d'émission.

Ainsi que cela a été mentionné, la cuve peut être, de manière connue en soi, cylindrique et a donc un diamètre donné ; dans ce cas, il est avantageux que les plaques portant les sources d'illumination aient une dimension radiale comprise entre 5% et 20% de ce diamètre, de préférence entre 7,5% et 15%, par exemple 10%.

La matière dans laquelle les sources d'illumination sont encapsulées est choisie en fonction des contraintes technologiques que l'on souhaite respecter. Ainsi, il s'agit de préférence d'une matière thermoplastique ayant à la fois de bonnes propriétés de transmission de la lumière et de la chaleur, en plus de sa compatibilité avec la masse à traiter. Ainsi, cette matière permet une bonne évacuation de la chaleur générée par les sources d'illumination vers la masse à traiter qui agit ainsi comme un puits de calories ; en effet, il est avantageux que la matière d'encapsulation soit capable d'évacuer dans la masse à traiter la chaleur générée par les sources d'illumination même lorsqu'il s'agit de LED. En alternative, des circuits d'évacuation de la chaleur (produite par les LED, ou produite par un procédé exothermique dans le réacteur) peuvent être prévus dans l'épaisseur des contre-pales. Il est en outre recommandé qu'une telle matière puisse résister à des températures élevées, entre 100°C et 150°C, telles que celles pouvant être utilisées lors des opérations de stérilisation ou de décontamination de la cuve. De telles opérations de stérilisation ou de décontamination peuvent être effectuées sur les plaques ou contre-pales indépendamment de la cuve lorsque ces plaques ou contre-pales sont amovibles.

Lorsque la masse à traiter est formée de micro-organismes, la matière d'encapsulation des sources d'illumination est de préférence totalement inerte par rapport au produit sans relargage, de manière à éviter tout risque de contamination de la masse par cette matière d'encapsulation.

Une matière particulièrement bien adaptée est le polysulfone qui combine une bonne compatibilité avec les normes alimentaires (y compris les normes américaines de la Food and Drug Administration, ou FDA en abrégé), un bon coefficient de transfert thermique (permettant une évacuation de la chaleur vers la masse de micro-organismes et un caractère semi-transparent permettant une bonne transmission de la lumière si le matériau a une épaisseur choisie entre 1 mm et 5 cm ; en outre ce matériau conserve ses propriétés après un éventuel traitement thermique de stérilisation ou de nettoyage avec des détergents ou de l'acide. Si l'on modifie la combinaison de caractéristiques souhaitées, d'autres matériaux peuvent être choisis, par exemple le polyuréthane, le polypropylène, un matériau acrylique ou un polycarbonate.

En pratique, les plaques portant la pluralité de sources d'illumination sont de préférence en un nombre compris entre 4 et 10 en étant régulièrement réparties autour de l'ensemble tournant ; toutefois, un nombre inférieur de plaques peut être en service à un instant donné.

Des objets, caractéristiques et avantages de l'invention ressortent de la description qui suit, donnée à titre d'exemple illustratif non limitatif, en regard du dessin annexé sur lequel :
- la figure 1 est un schéma en coupe axiale d'un réacteur conforme à l'invention,
- la figure 2 est un schéma en coupe transversale de ce réacteur, et
- la figure 3 est une vue de détail d'un exemple de plaque comportant une pluralité de sources d'illumination.

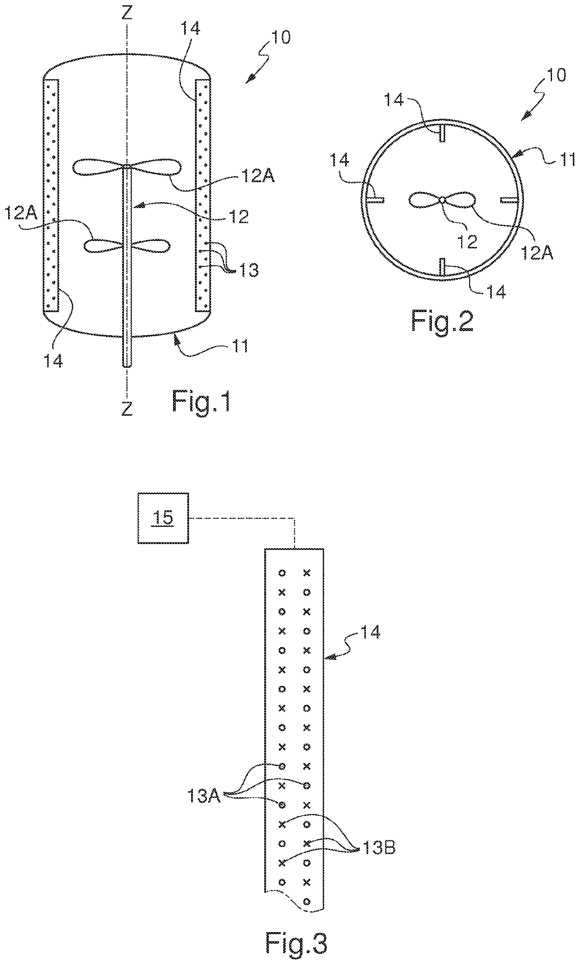

Les figures 1 et 2 représentent de manière schématique un réacteur conforme à l'invention ; il est ici décrit à propos du traitement d'une masse à traiter constituée d'une biomasse formée de micro-organismes, par exemple des micro-algues ; on comprend toutefois que la description qui suit s'applique également à d'autres types de réacteurs, chimiques ou physico-chimiques. Ce bioréacteur, ou fermenteur, est noté 10 dans son ensemble ; il comporte principalement une cuve 11 destinée à contenir une masse de micro-organismes (non représentée), un ensemble 12 tournant autour d'un axe Z-Z destiné à assurer un brassage de cette masse de micro-organismes et une pluralité de sources d'illumination 13 destinées à favoriser la croissance de cette masse de micro-organismes ; cette cuve 11 a une paroi interne à laquelle sont fixées des plaques 14 dont les plans sont orientés vers l'axe de l'ensemble tournant et parallèlement à celui-ci en sorte d'empêcher la formation d'un vortex au sein de la masse de micro-organismes sous l'action de l'ensemble tournant ; de telles plaques 14 sont couramment appelées contre-pales en référence aux pales 12A que comporte habituellement l'élément tournant.

Un tel réacteur peut, en fonction de son application, comporter d'autres éléments (non représentés) tels que, notamment, une voie d'entrée d'un produit à traiter ou à dégrader au moyen de la biomasse, ou une voie d'alimentation en réactif ou en éléments nutritifs tels que des sucres pour la prolifération de la biomasse, une voie d'arrivée et de sortie d'air ou de gaz, ou une voie de soutirage de micro-organismes.

Les sources d'illumination 13 sont portées par lesdites plaques en étant encapsulées, avec au moins la partie de ces plaques qui les portent, dans une matière (non représentée) compatible avec les micro-organismes et en une épaisseur permettant de diffuser ladite lumière vers l'intérieur de la cuve. De préférence, les sources d'illumination distribuées sur une face d'une telle plaque sont encapsulées par un matériau recouvrant la totalité de cette face.

La figure 3 représente, à titre d'exemple, un détail d'une telle plaque 14. On y voit un premier ensemble de sources d'illumination 13A schématisées sous la forme de petits cercles et disposées en deux colonnes telles qu'une source d'une colonne est disposée à un niveau intermédiaire entre ceux des sources les plus proches dans l'autre colonne. Il peut bien entendu y avoir un plus grand nombre de colonnes, et les sources peuvent être disposées en quinconce ou en sorte de former des groupes rectangulaires.

Dans l'exemple représenté, on observe en outre un second ensemble de sources d'illumination 13B schématisées sous la forme de petites croix, disposées selon une configuration similaire à celle du premier ensemble ; les sources de ce second ensemble sont ici disposées en sorte d'alterner avec les sources du premier ensemble, c'est-à-dire qu'elles sont disposées en deux colonnes superposées, perpendiculairement au plan de la figure 3, les sources d'une colonne d'un ensemble alternant avec les sources de la colonne correspondante de l'autre ensemble. En variante, les colonnes du second ensemble peuvent alterner avec les colonnes de l'autre ensemble et peuvent être en un nombre supérieur à deux, éventuellement différent du nombre de colonnes du premier ensemble.

Les sources d'illumination sont ici distribuées sur les deux faces des plaques, les sources 13A étant situées sur l'une des faces tandis que les sources 13B sont situées sur la face opposée.

En effet, il peut y avoir, selon les besoins en illumination, des sources d'un seul côté d'une plaque donnée, ou au contraire des sources réparties sur les deux faces d'une telle plaque. Les sources situées sur une face peuvent être réparties en fonction de la répartition des sources sur l'autre face, en superposition ou au contraire en sorte de se compléter ; il peut aussi y avoir des sources disposées sur les deux faces indépendamment l'une de l'autre.

Lorsqu'il y a des sources d'illumination sur les deux faces de la plaque, le matériau d'encapsulation englobe avantageusement la plaque sur ses deux faces, ce qui favorise une bonne tenue du matériau sur la plaque. Pour la même raison de bonne tenue mécanique, on comprend que, même lorsque les sources ne sont disposées que sur une face, il peut être avantageux que le matériau englobe aussi bien la face portant des sources que l'autre face restant nue.

De manière préférée, les sources d'illumination sont des diodes électroluminescentes, aussi appelées LED en abrégé. De telles sources présentent notamment un faible encombrement, une faible consommation électrique tout en générant une faible quantité de chaleur, par rapport aux autres sources d'illumination connues ; lorsqu'il s'agit de LED organiques (aussi appelées OLED), leur épaisseur peut être très faible. En variante, ces sources d'illumination peuvent être constituées par les extrémités de groupes de fibres optiques s'étendant depuis une (ou plusieurs) source(s) disposée(s) à l'extérieur de la cuve.

Les sources d'illumination peuvent être toutes identiques en ayant un même régime d'excitation ; on comprend toutefois que, en variante, les sources d'illumination contenues dans la cuve peuvent être réparties en plusieurs sous-ensembles, chaque sous-ensemble pouvant contenir une seule source d'illumination ou plusieurs sources d'illumination avantageusement réparties de manière homogène, chaque sous-ensemble étant piloté individuellement selon plusieurs paramètres opératoires : éclairage en tout ou rien, ou éclairage continu avec une intensité éventuellement variable, Il peut toutefois être plus simple d'affecter à chaque sous-ensemble un spectre d'émission spécifique, par exemple un sous-ensemble émettant de la lumière blanche, un autre sous-ensemble émettant dans le bleu, un autre sous-ensemble émettant dans le rouge, etc. ; selon les besoins, l'opérateur peut alors choisir de n'exciter que les sous-ensembles qui sont appropriés pour le régime d'illumination souhaité.

En pratique, il est toutefois avantageux d'utiliser l'ensemble des sources disponibles pour obtenir une illumination suffisante.

Les principaux régimes possibles d'excitation des sources d'illumination sont :
- un régime flash à une fréquence pouvant aller par exemple de 1 à 150 kHz,
- un régime continu, ayant éventuellement des variations lentes de manière à simuler une illumination naturelle).

Quel que soit le régime, s'il y a des variations, on peut prévoir des cycles allant de 1 s à 24 h.

Ces divers régimes consistent à alimenter les sources en contrôlant leur excitation, à un niveau choisi en fonction des besoins, entre 0% et 100% de leur puissance nominale.

Ces sources sont en pratique choisies pour avoir un pouvoir d'illumination global compris entre 1 et 3000 micro-Einstein. Une telle gamme permet d'englober des traitements chimiques et de stérilisation.

On comprend que dans le cas de bioréacteurs pour la croissance de micro-algues, on peut ainsi, selon les besoins, notamment simuler l'un ou l'autre des régimes autotrophe, ou mixotrophe.

Ainsi, on peut prévoir une décomposition homogène dans les types de sources d'illumination ou au contraire plusieurs décompositions de la pluralité des sources d'illumination, éventuellement combinées.

Selon un premier exemple, la pluralité de sources d'illumination peut être composée de plusieurs sous-ensembles, les sources d'illumination de chacun de ces sous-ensembles ayant un spectre spécifique d'émission avec une intensité constante ou variable.

Selon un second exemple, la pluralité de sources d'illumination est composée de plusieurs sous-ensembles ayant chacun un même régime d'excitation, en continu ou de manière cyclique, avec une intensité constante ou variable.

Dans un troisième exemple, la pluralité de sources d'illumination est connectée à un ensemble de pilotage tel que certaines au moins des sources d'illumination peuvent être pilotées en mode d'éclairage ou non, en régime flash, continu avec ou sans cycle.

Malgré ces diverses possibilités, on comprend toutefois que l'option consistant à avoir simplement sur une plaque des sources d'illumination d'un type unique est d'un grand intérêt pratique.

Un réacteur conforme à l'invention a une grande flexibilité de fonctionnement et donc une grande polyvalence.

Les contre-pales équipées de sources d'illumination sont avantageusement identiques en sorte d'être interchangeables ; il peut toutefois y avoir des contre-pales avec sources et des contre-pales sans sources d'illumination.

La densité de sources d'illumination par unité de surface de plaque contre-pale peut être choisie en fonction des besoins (dans certaines applications, cette densité peut être variable, réduite voire nulle aux extrémités et maximale à mi-hauteur ; en variante, il n'y a des sources d'illumination que sur une partie de la hauteur des plaques, par exemple à l'exclusion des extrémités) ; lorsque ces sources sont des OLED, celles-ci peuvent être disposées en sorte d'être sensiblement adjacentes ; en pratique, les sources d'illumination sont réparties de manière régulière avec un pas qui peut être choisi avec une grande liberté, dans la gamme allant d'à peine quelques millimètres (par exemple 0.5 cm) jusqu'à de l'ordre d'un mètre (en hauteur et/ou en largeur).

Le fonctionnement d'un réacteur fait intervenir d'autres paramètres opératoires tels que la température, le pH, la teneur en oxygène dissous, la vitesse d'agitation, la pression, etc. Toutefois, ces paramètres n'étant pas modifiés par la configuration des sources d'illumination selon l'invention, ils ne sont pas détaillés ici.

Surtout lorsqu'il s'agit de LED organiques, les sources peuvent être fixées aux contre-pales par simple collage.

De manière classique en soi, la cuve et les contre-pales sont habituellement en acier inox ou équivalent mais d'autres matériaux peuvent être choisis en fonction des besoins.

De manière connue en soi, la cuve est avantageusement cylindrique. Si D est son diamètre et H sa hauteur, les plaques portant les sources d'illumination ont de préférence une dimension radiale comprise entre 7,5% et 15%, par exemple de l'ordre de 10% (typiquement entre 9% et 11%). Cela est compatible avec la fonction de contre-pale assurée par ces plaques.

De manière particulièrement avantageuse, les plaques portant les sources d'illumination sont démontables vis-à-vis de la paroi de la cuve. Il en résulte une grande facilité de maintenance, mais aussi une grande facilité pour adapter une cuve donnée à une application donnée, en choisissant des plaques ayant la bonne densité de sources d'illumination, ou en faisant varier le nombre de plaques portant de telles sources d'illumination.

Dans l'exemple des figures 1 et 2, la cuve est munie de quatre plaques 14 servant de contre-pales. On comprend que ce nombre peut être modifié ; il peut être plus faible (une seule contre-plaque permet déjà d'assurer un effet de contre-pale, au moins partiel), voire plus important ; il est toutefois intéressant de le choisir dans la plage de 2 à 10 (par exemple 4 à 10) étant précisé que ce nombre peut être d'autant plus important que le diamètre de la cuve augmente pour conserver le rôle d'anti-vortex. Ces plaques sont en pratique régulièrement réparties autour de l'ensemble tournant.

De préférence, toutes les plaques servant de contre-pales sont équipées de sources d'illumination, mais on peut prévoir qu'une partie seulement des plaques contre-pales servent de support à des sources d'illumination ; cela dépend des utilisations.

La matière dans laquelle les sources d'illumination sont encapsulées sert à protéger ces sources vis-à-vis des micro-organismes que la cuve peut être amenée à contenir, tout en favorisant la solidarisation au cours du temps de ces sources à leur plaque de support. Le choix de cette matière dépend des utilisations possibles de la cuve considérée ; de manière générale, cette matière doit être compatible avec les micro-organismes tout en permettant une bonne diffusion vers l'extérieur de la lumière générée par les sources et, de préférence, une bonne diffusion de chaleur ; on comprend toutefois que cette capacité dépend non seulement du matériau mais aussi de l'épaisseur de matière qui recouvre les sources (dès lors que le rôle d'encapsulation peut être obtenu avec une faible épaisseur, il n'est pas nécessaire que le matériau soit intrinsèquement transparent ; il peut n'être que partiellement transparent).

Dans le cas de la fermentation de micro-algues, il est apparu que le polysulfone était un matériau particulièrement approprié, étant compatible avec les micro-algues au sens des normes alimentaires, étant semi-transparent, et étant capable de résister à des traitements de stérilisation à haute température et de nettoyage aux détergents et à l'acide ; on comprend toutefois que d'autres matériaux peuvent être choisis en fonction des propriétés recherchées.

Il est intéressant de noter que, en jouant sur les deux paramètres que sont la largeur des contre-pales par rapport au diamètre de la cuve et le nombre de ces contre-pales, on peut obtenir une surface d'illumination par unité de volume qui reste sensiblement constant au sein d'une large plage de volume, ainsi que cela ressort du tableau suivant, où :
* « V » désigne le volume utile de la cuve (en m³),
* « H » désigne la hauteur de la virole (en m),
* « Di » désigne le diamètre interne de la cuve (en m),
« CP » désigne les contre-pales,
* « CP/Di » désigne le rapport entre la dimension radiale des contre-pales et le diamètre de la cuve,
* « S(CP) » désigne la surface totale des deux faces d'une contre-pale (en m²),
* « N (CP) » désigne le nombre de contre-pales équipées de sources d'illumination,
* « Stot » désigne la surface totale des contre-pales (en m²)
* « Scp/V » désigne la surface d'illumination par unité de volume de la cuve (en m²/m³).

On observe que, en laissant varier le rapport CP/Di entre 7% et 13%, et en faisant varier le nombre de contre-pales équipées de sources d'illumination entre 1 (pour des cuves prototypes) et 10, la surface d'illumination par unité de volume peut être maintenue dans la plage de 0,75m²/m³+/-0,05m²/m³.

| | **Exemple d'une cuve d'un ratio H / Di de 2,0** | | | | | | | | | | |
|---|---|---|---|---|---|---|---|---|---|---|---|
| **V (m3)** | **0,1** | **0,2** | **0,5** | **1** | **2** | **5** | **10** | **15** | **20** | **50** | **100** |
| **H (m)** | 0,975 | 1,215 | 1,618 | 2,029 | 2,551 | 3,408 | 4,265 | 4,851 | 5,33 | 7,188 | 9,031 |
| **Di (m)** | 0,404 | 0,509 | 0,69 | 0,89 | 1,097 | 1,489 | 1,874 | 2,147 | 2,362 | 3,204 | 4,034 |
| **Ratio CP/Di** | 0,095 | 0,12 | 0,09 | 0,1 | 0,09 | 0,12 | 0,12 | 0,11 | 0,1 | 0,1 | 0,13 |
| **S(CP)** | 0,0748 | 0,1484 | 0,2009 | 0,3611 | 0,5037 | 1,2178 | 1,9182 | 2,2913 | 2,5178 | 4,606 | 9,472 |
| **N (CP)** | 1 | 1 | 2 | 2 | 3 | 3 | 4 | 5 | 6 | 8 | 8 |
| **Stot (m2)** | 0,0748 | 0,1484 | 0,4019 | 0,722 | 1,5111 | 3,653 | 7,672 | 11,456 | 15,107 | 36,848 | 75,776 |
| **Scp/V (m2/m3)** | **0,748** | **0,742** | **0,804** | **0,722** | **0,756** | **0,731** | **0,767** | **0,764** | **0,755** | **0,737** | **0,758** |

On appréciera que l'invention s'applique à des bioréacteurs et fermenteurs pouvant avoir une grande variété.

Ainsi ces bioréacteurs peuvent être des réacteurs de bio-production, de production de biomasse de micro-organismes photosynthétiques, ou hétérotrophes ou mixotrophes de photocatalyse, de traitement d'effluents, par exemple pour le traitement d'eaux usées.

Les micro-organismes peuvent contenir des chloroplastes ou non. Il a pu être constaté pour ceux qui n'en contiennent pas qu'ils produisent des pigments via des photorécepteurs.

Les micro-organismes peuvent être notamment des bactéries, des champignons, des micro-algues, etc.

Toutefois les commentaires qui précèdent s'appliquent également à ces réacteurs de traitement physique, ou physico-chimique, tels que des réacteurs de stérilisation, ou de décontamination, ou encore de traitement par catalyseur sensible à la lumière.

Les sources de lumière peuvent être des LED (système avec une ou plusieurs longueurs d'onde), avec par exemple des LED bleues et rouges assurant une illumination en alternance, des fibres optiques, des lampes UV, etc. Le spectre d'illumination peut en effet être en dehors du spectre visible.

La cuve représentée a un axe de symétrie disposé verticalement, mais on comprend que les commentaires ci-dessus peuvent se généraliser dans le cas d'une cuve ayant un axe incliné, les notions de hauteur et de largeur pouvant alors être remplacées par des notions de dimension longitudinale, ou axiale, et de dimension transversale (il est rappelé que la cuve peut ne pas être cylindrique).

On comprend qu'un intérêt de la configuration décrite est que celle-ci est transposable à une échelle différente en conservant à peu près constant le rapport de la surface d'illumination par rapport au volume de la cuve, l'ensemble étant résistant à des opérations de stérilisation ou de nettoyage en place lorsqu'il s'agit d'un bioréacteur ou d'un fermenteur.

## Revendications

1. Réacteur (10) comportant une cuve (11) destinée à contenir une masse à traiter et munie d'une part d'un ensemble (12) tournant autour d'un axe (Z-Z) destiné à assurer un brassage de cette masse à traiter et d'autre part d'une pluralité de sources d'illumination (13, 13A, 13B) destinée à favoriser le traitement de cette masse, cette cuve ayant une paroi interne à laquelle sont fixées des plaques (14) dont les plans sont orientés vers l'axe de l'ensemble tournant et parallèlement à celui-ci en sorte d'empêcher la formation d'un vortex au sein de la masse à traiter sous l'action de l'ensemble tournant, ces sources d'illumination étant portées par lesdites plaques en étant encapsulées, avec au moins la partie de ces plaques qui les porte, dans une matière compatible avec la masse à traiter et en une épaisseur permettant de diffuser ladite lumière vers l'intérieur de la cuve.

2. Réacteur selon la revendication 1, dans lequel la pluralité de sources d'illumination comporte des sources d'illumination (13A, 13B) distribuées sur chacune des faces desdites plaques.

3. Réacteur selon la revendication 1 ou la revendication 2, dans lequel les plaques (14) portant les sources d'illumination sont démontables vis-à-vis de la paroi de la cuve.

4. Réacteur selon l'une quelconque des revendications 1 à 3, dans lequel les sources d'illumination (13, 13A, 13B) sont des diodes électro-luminescentes.

5. Réacteur selon l'une quelconque des revendications 1 à 4, dans lequel la pluralité de sources d'illumination est composée de plusieurs sous-ensembles, les sources d'illumination de chacun de ces sous-ensembles ayant un spectre spécifique d'émission.

6. Réacteur selon l'une quelconque des revendications 1 à 5, dans lequel la pluralité de sources d'illumination est connectée à un ensemble de pilotage tel que certaines au moins des sources d'illumination peuvent être pilotées en mode d'éclairage ou non, en régime flash, en régime continu ou de manière cyclique.

7. Réacteur selon l'une quelconque des revendications 1 à 6, dans lequel la cuve est cylindrique en ayant un diamètre donné, les plaques portant les sources d'illumination ayant une dimension radiale comprise entre 5% et 20% de ce diamètre.

8. Réacteur selon l'une quelconque des revendications 1 à 7, dans lequel la matière dans laquelle les sources d'illumination sont encapsulées est une matière thermoplastique choisie en sorte de permettre une bonne évacuation de la chaleur générée par ces sources d'illumination.

9. Réacteur selon l'une quelconque des revendications 1 à 8, dans lequel la matière thermoplastique dans laquelle les sources d'illumination sont encapsulées est du polysulfone.

10. Réacteur selon l'une quelconque des revendications 1 à 9, dans lequel les plaques portant la pluralité de sources d'illumination sont au nombre de 1 à 10 en étant, lorsque le nombre est supérieur à 2, régulièrement réparties autour de l'ensemble tournant.

## Patentansprüche

1. Reaktor (10), aufweisend einen Behälter (11), der bestimmt ist, eine zu behandelnde Masse zu enthalten und zum einen mit um eine Achse (Z-Z) drehenden Einheit (12), die bestimmt ist, ein Rühren dieser zu behandelnden Masse zu gewährleisten, und zum anderen mit einer Vielzahl von Lichtquellen (13, 13A, 13B), die bestimmt ist, die Behandlung dieser Masse zu fördern, ausgestattet ist, wobei dieser Behälter eine Innenwand hat, an der Platten (14) befestigt sind, deren Ebenen zur Achse der drehenden Einheit ausgerichtet sind und parallel zu dieser zeigen, um die Bildung eines Wirbels innerhalb der zu behandelnden Masse unter der Einwirkung der drehenden Einheit zu verhindern, wobei diese Lichtquellen von den Platten getragen werden, indem sie mit mindestens dem Teil dieser Platten, der sie trägt, in ein Material, das mit der zu behandelnden Masse kompatibel ist und in eine Dicke, die erlaubt, das Licht im Innern des Behälters zu verteilen, eingekapselt sind.

2. Reaktor nach Anspruch 1, wobei die Vielzahl von Lichtquellen auf jeder der Seiten der Platten verteilte Lichtquellen (13A, 13B) aufweist.

3. Reaktor nach Anspruch 1 oder Anspruch 2, wobei die die Lichtquellen tragenden Platten (14) in Bezug zur Wand des Behälters demontierbar sind.

4. Reaktor nach einem der Ansprüche 1 bis 3, wobei die Lichtquellen (13, 13A, 13B) elektrolumineszierende Dioden sind.

5. Reaktor nach einem der Ansprüche 1 bis 4, wobei die Vielzahl von Lichtquellen von mehreren Untereinheiten zusammengesetzt ist, wobei die Lichtquellen jeder dieser Untereinheiten ein spezielles Sendespektrum haben.

6. Reaktor nach einem der Ansprüche 1 bis 5, wobei die Vielzahl von Lichtquellen mit einer Steuereinheit verbunden ist, so dass mindestens einige der Lichtquellen im Beleuchtungsmodus oder nicht, im Blitzbetrieb, im kontinuierlichen Betrieb oder zyklisch steuerbar sind.

7. Reaktor nach einem der Ansprüche 1 bis 6, wobei der Behälter zylindrisch ist, indem er einen bestimmten Durchmesser hat, wobei die die Lichtquellen tragenden Platten eine radiale Abmessung zwischen 5% und 20% dieses Durchmessers haben.

8. Reaktor nach einem der Ansprüche 1 bis 7, wobei das Material, in welches die Lichtquellen eingekapselt sind, ein thermoplastisches Material ist, das derart ausgewählt ist, dass eine gute Ableitung der von diesen Lichtquellen erzeugten Wärme gestattet ist.

9. Reaktor nach einem der Ansprüche 1 bis 8, wobei das thermoplastische Material, in welches die Lichtquellen eingekapselt sind, Polysulfon ist.

10. Reaktor nach einem der Ansprüche 1 bis 9, wobei die die Vielzahl von Lichtquellen tragenden Platten in der Anzahl von 1 bis 10 sind und dabei, wenn die Anzahl größer als 2 ist, regelmäßig um die drehende Einheit verteilt sind.

## Claims

1. A reactor (10) comprising a tank (11) configured to contain a mass to treat and provided with an assembly (12) turning around an axis (Z-Z) configured to provide mixing of that mass to treat and further provided with a plurality of illumination sources (13, 13A, 13B) configured to promote the treatment of that mass, said tank having an inside wall to which are fastened plates (14) the planes of which are oriented towards the axis of the turning assembly and parallel thereto, so as to prevent the formation of a vortex within the mass to treat under the action of the turning assembly, said illumination sources being carried by said plates and being encapsulated, with at least the part of those plates which carries them, in a material compatible with the mass to treat and having a thickness enabling said light to be diffused towards the inside of the tank.

2. The reactor according to claim 1, wherein the plurality of illumination sources comprises illumination sources (13A, 13B) distributed on each of the faces of said plates.

3. The reactor according to claim 1 or claim 2, wherein the plates (14) carrying the illumination sources are demountable from the wall of the tank.

4. The reactor according to any one of claims 1 to 3, wherein the illumination sources (13, 13A, 13B) are light-emitting diodes.

5. The reactor according to any one of claims 1 to 4, wherein the plurality of illumination sources is composed of several sub-assemblies, the illumination sources of each of those sub-assemblies having a specific emission spectrum.

6. The reactor according to any one of claims 1 to 5, wherein the plurality of illumination sources is connected to an operating assembly such that at least some of the illumination sources may be operated in lighting or non-lighting mode, in flash mode, in continuous mode or cyclically.

7. The reactor according to any one of claims 1 to 6, wherein the tank is cylindrical while having a given diameter, the plates carrying the illumination sources having a radial dimension comprised between 5% and 20% of that diameter.

8. The reactor according to any one of claims 1 to 7, wherein the material in which the illumination sources are encapsulated is a thermoplastic material chosen so as to enable good evacuation of the heat generated by these illumination sources.

9. The reactor according to any one of claims 1 to 8, wherein the thermoplastic material in which the illumination sources are encapsulated is polysulfone.

10. The reactor according to any one of claims 1 to 9, wherein the plates carrying the plurality of illumination sources are from 1 to 10 in number and, when the number is greater than 2, are regularly distributed around the turning assembly.
